(19) Europäisches Patentamt
European Patent Office
Office européen des brevets

(11) Numéro de publication : 0 290 321 B1

# (12) FASCICULE DE BREVET EUROPEEN

(45) Date de publication du fascicule du brevet : **02.10.91 Bulletin 91/40**

(51) Int. Cl.5 : **C07H 15/203,** C07C 323/09, C07C 323/19, C07C 323/31, A61K 31/70

(21) Numéro de dépôt : **88401030.7**

(22) Date de dépôt : **27.04.88**

## (54) Nouveaux beta-D-phényl-thioxylosides, leur procédé de préparation et leur application en thérapeutique.

(30) Priorité : **04.05.87 FR 8706237**

(43) Date de publication de la demande : **09.11.88 Bulletin 88/45**

(45) Mention de la délivrance du brevet : **02.10.91 Bulletin 91/40**

(84) Etats contractants désignés : **AT BE CH DE ES FR GB GR IT LI LU NL SE**

(56) Documents cités :
**EP-A- 0 051 023**
**EP-A- 0 133 103**
**US-A- 3 243 425**

(73) Titulaire : **FOURNIER INDUSTRIE ET SANTE**
**38, avenue Hoche**
**F-75008 Paris (FR)**

(72) Inventeur : **Samreth, Soth**
**2, rue de la 2ème Escadre**
**F-21600 Longvic (FR)**
Inventeur : **Bellamy, François**
**rue Basse Cédex 11 Saulon la Rue**
**F-21910 Saulon La Chapelle (FR)**
Inventeur : **Millet, Jean**
**15 rue du Tremblois Corcelles les citeaux**
**F-21910 Saulon La Rue (FR)**

(74) Mandataire : **Clisci, Serge et al**
**S.A. FEDIT-LORIOT & AUTRES 38, Avenue Hoche**
**F-75008 Paris (FR)**

EP 0 290 321 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne, en tant que produits industriels nouveaux, les dérivés de β-D-phényl-thioxylosides de formule I ci-dessous. Elle concerne également leur procédé de préparation et leur application en thérapeutique, en tant qu'agents antithrombotiques, notamment antithrombotiques veineux.

On a déjà proposé dans EP-B-0051023 des dérivés de benzoyl- et d'α-hydroxybenzyl-phényl-osides en tant qu'agents anti-ulcéreux, anti-agrégants plaquettaires, antithrombotiques et oxygénateurs cérébraux.

On connait également de EP-A-0133103 des benzyl-phényl-osides utiles un tant qu'agents hypocholestérolémiants et hypolipidémiants, certains de ces composés, en particulier le produit de l'exemple 1, présentant en outre des effets antithrombotiques.

On vient à présent de trouver que les dérivés de β-D-phényl-thioxylosides selon l'invention, structurellement différents des produits connus de l'art antérieur, sont utiles dans le traitement et la prévention des maladies liées aux troubles circulatoires, notamment en tant qu'agents antithrombotiques veineux.

Les dérivés selon l'invention présentent, de façon inattendue, des propriétés antithrombotiques très supérieures à celles des produits connus de l'art antérieur, voir à cet effet les résultats des essais comparatifs consignés dans le tableau III ci-après.

Les nouveaux produits selon l'invention sont caractérisés en ce qu'ils sont choisis parmi l'ensemble constitué par :

(i) les β-D-phényl-thioxylosides de formule :

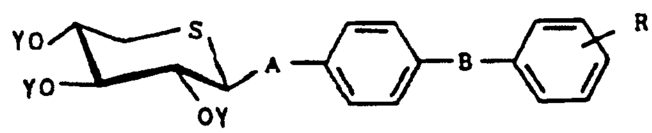

(I)

dans laquelle :

R représente un atome d'hydrogène, un atome d'halogène, un groupe nitro ou un groupe cyano,

A représente l'atome de soufre ou l'atome d'oxygène,

B représente un groupe $CH_2$, CHOH ou CO,

Y représente l'atome d'hydrogène ou un groupe acyl ; et,

(ii) leurs épimères quand B est CHOH.

Les fonctions hydroxyle du reste β-D-thioxylose sont susceptibles d'être acylées, notamment acétylées. La présente invention comprend donc les dérivés de formule I où les fonctions hydroxyle du reste β-D-thioxylose sont acylées, notamment acétylées.

Parmi les atomes d'halogène intervenant dans la définition du groupe R, on peut citer les atomes de fluor, de chlore et de brome, l'atome d'halogène préféré étant l'atome de chlore.

Parmi les groupes acyles qui conviennent selon l'invention, on peut mentionner ceux qui renferment au total 2 à 5 atomes de carbone, le groupe acyle préféré étant $CH_3CO$.

Les composés de formule I et les composés acylés corespondants peuvent être préparés selon une réaction de glycosidation caractérisée en ce que :

(i) on fait réagir un composé de formule :

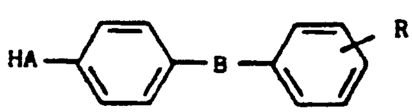

(II)

où A, B et R sont définis comme ci-dessus,

avec un dérivé du thioxylose choisi parmi l'ensemble comprenant les haloacylthioxylosides et les acylthioxylosides de formules :

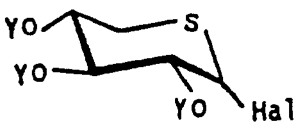

(VIIIa)

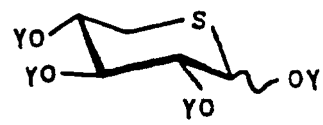

(VIIIb)

dans lesquelles Hal représente un atome d'halogène tel que Cl ou Br (l'atome de brome étant ici l'atome d'halogène préféré) et Y représente un groupe acyle, notamment un groupe acyle aliphatique comprenant un nombre total d'atomes de carbone de 2 à 5 et de préférence le groupe acétyle :
dans un solvant inerte, à raison de 1 mole de II pour environ 1,1 à 1,2 moles de dérivé du thioxylose, en présence d'un accepteur d'acide ou d'un acide de Lewis et,
(ii) si nécessaire, on procède à une réaction de désacylation à une température comprise entre la température ambiante (15-25°C) et la température de reflux du milieu réactionnel, dans un alcool inférieur en $C_1$-$C_4$ (de préférence le méthanol), en présence d'un alcoolate métallique (de préférence le méthylate de magnésium ou le méthylate de sodium).

Dans ce procédé, il est important au stade (i) que le composé VIIIa soit de configuration α. En revanche, le composé VIIIb peut être de configuration α ou β ou encore un mélange des deux configurations.

On peut aussi obtenir les composés, acylés ou non, de formule I dans laquelle B représente soit CHOH soit CH2, par réduction selon une méthode connue en soi des composés de formule I (acylés ou non) dans laquelle B représente CO ou CHOH.

On peut encore obtenir les composés, acylés ou non, de formule I où B représente CO, par oxydation selon une méthode connue en soi des composés de formule I (acylés ou non) où B représente $CH_2$ ou CHOH.

Parmi les méthodes de glycosidation connues de l'homme de l'art, on préconise :

— la méthode de KOENIGS-KNORR (décrite dans "The Carbohydrates, Chemistry and Biochemistry", 2nd Edition, New York and London : Academic Press (1972), tome IA, pages 295-301), par condensation d'un phénol, ou d'un thiophénol de formule II avec un haloacylthioxyloside VIIIa, dans un solvant inerte choisi parmi les solvants polaires et apolaires (comme par exemple le diméthylformamide, le tétrahydrofuranne, le dioxanne, l'acétonitrile, le nitrométhane, le benzène, le toluène, les xylènes et leurs mélanges), en présence d'un accepteur de proton tel que le cyanure mercurique ou le triflate d'argent (trifluorométhyl-sulfonate d'argent) ; et,

— la méthode de HELFERICH (ibidem, pages 292-294) par condensation d'un acylthioxyloside VIIIb avec un phénol ou un thiophénol de formule II, dans un solvant inerte choisi parmi les solvants aromatiques, les solvants chlorés, les éthers et leurs mélanges, en présence d'un acide de Lewis.

Selon un mode préféré de mise en oeuvre de l'invention, on préconise, lorsque A représente un atome de soufre dans le composé de formule II, au stade (i) du procédé, de condenser 1 mole du thiol II avec environ 1,1 à 1,2 moles d'haloacylthioxyloside VIIIa dans un solvant inerte choisi parmi les solvants polaires et apolaires, en présence de cyanure mercurique.

On utilisera avantageusement le 2,3,4-tri-O-acétyl-1-bromo-α-D-5-thioxylopyranoside dans un mélange benzène/nitrométhane 1/1 (v : v), en présence de 1,1 à 1,3 moles de cyanure mercurique, à une température comprise entre 0°C et la température de reflux du millieu réactionnel, de préférence à environ 40-50°C, pendant 1 à 4 heures, de préférence pendant environ 2 heures.

Selon un autre mode préféré de mise en oeuvre de l'invention, on préconise, lorsque A représente un atome d'oxygène et B représente le groupe méthylène dans le composé de formule II, au stade (i) du procédé, de condenser 1 mole du phénol II avec environ 1,1 à 1,2 moles d'haloacylthioxyloside VIIIa, dans un solvant inerte choisi parmi les solvants aromatiques, les solvants chlorés, les éthers et leurs mélanges, en présence de triflate d'argent.

On utilisera avantageusement le 2,3,4-tri-O-acétyl-1-bromo-α-D-5-thioxylopyranoside dans un mélange anhydre toluène/nitrométhane 1/1 (v : v), en présence de 1,1 à 1,3 moles de triflate d'argent, la réaction étant effectuée à l'abri de la lumière, à une température comprise entre 0°C et 15°C de préférence à environ 3°C, pendant 5 à 24 heures, de préférence pendant environ 12 heures.

Selon un autre mode préféré de mise en oeuvre de l'invention, on préconise également, lorsque A représente un atome de soufre dans le composé de formule II, au stade (i) du procédé, de condenser 1 mole du thiol II avec environ 1,1 à 1,3 moles d'acylthioxyloside VIIIb dans un solvant inerte choisi parmi les éthers, les solvants aromatiques, les solvants chlorés et leurs mélanges, en présence de $SnCl_4$.

On utilisera avantageusement le 1,2,3,4-tétra-O-acétyl- α ou β-D-5-thioxylopyranoside, dans le chlorure

de méthylène, en présence de 1,1 à 1,2 moles de $SnCl_4$ à une température comprise entre 0°C et la température de reflux du milieu réactionnel, de préférence à environ 20°C, pendant 1 à 5 heures, de préférence pendant environ 3 heures.

La réaction de glycosidation conduit dans tous les cas à un mélange des isomères α et β en proportions variables.

L'isomère β est isolé selon les méthodes connues de l'homme de l'art, comme par exemple la cristallisation fractionnée ou la chromatographie, notamment la "flash chromatography" : chromatographie sur colonne de silice et sous pression selon la technique décrite par W.C. STILL et al. dans J. Org. Chem. (1978), 42 (N° 14) 2923.

Les réactions de réduction qui permettent d'obtenir les composés, acylés ou non, de formule I dans laquelle B est CHOH, à partir des composés correspondants où B est CO, mettent en oeuvre des réactifs classiques comme les hydrures métalliques tels que $LiAlH_4$, $KBH_4$ ou $NaBH_4$, dans des solvants inertes tel que l'éther, le tétrahydrofuranne ou les alcools inférieurs, notamment le méthanol et l'éthanol à une température comprise entre 0°C et la température ambiante (15°-25°C), pendant 1 à 12 heures, l'hydrure métallique préféré étant $NaBH_4$, la réaction étant conduite de préférence dans le méthanol à une température de 20°C.

Les réactions de réduction qui permettent d'obtenir les composés, acylés ou non, de formule I dans laquelle B est $CH_2$, à partir des composés correspondants où B est CO ou CHOH mettent en oeuvre des agents réducteurs comme les hydrures métalliques tels que $NaBH_4$ ou $KBH_4$ et de préférence $NaBH_4$, dans l'acide trifluoroacétique. Ici le meilleur mode de mise en oeuvre consiste à introduire l'agent réducteur dans le mélange contenant le composé à réduire et l'acide trifluoroacétique, à une température comprise entre la température de solidification du milieu réactionnel et 0°C, de préférence à 0°C, à raison d'un excès d'agent réducteur par rapport au composé à réduire, et, lorsque l'addition de l'agent réducteur est terminée, laisser réagir sous agitation pendant 0,5 à 12 heures, à une température comprise entre 0°C et 20°C. En pratique, il est avantageux, pour solubiliser le composé à réduire, d'associer l'acide trifluoroacétique à un solvant chloré, notamment le chlorure de méthylène.

Les réactions d'oxydation qui permettent d'obtenir les composés, acylés ou non, de formule I dans laquelle B est CO, à partir des composés correspondants où B est $CH_2$, mettent en oeuvre des oxydants classiques comme $CuSO_4/K_2S_2O_8$ ou $Cr_2O_3$, en présence d'une base organique comme la pyridine, dans un solvant polaire ou apolaire, tel que les éthers, les solvants aromatiques, les solvants chlorés et leurs mélanges, et, de préférence, le mélange eau/acétonitrile 1/1 (v : v) lorsqu'on utilise $CuSO_4/K_2S_2O_8$, et le chlorure de méthylène lorsqu'on utilise $Cr_2O_3$.

Les dérivés obtenus sont soumis, le cas échéant, à une désacylation, plus particulièrement à une désacétylation, qui est réalisée à une température comprise entre la température ambiante et la température de reflux du milieu réactionnel dans un alcool inférieur en $C_1$-$C_4$, en présence de l'alcoolate métallique correspondant. De préférence, on choisira le méthanol comme alcool inférieur et le méthanolate de sodium ou de magnésium comme alcoolate métallique.

Les réactions de désacylation et de réduction (en particulier passsage du CO au CHOH) peuvent éventuellement être conduites successivement sans isoler le composé intermédiaire formé.

Les dérivés intermédiaires de formule II dans laquelle A représente un atome de soufre sont des composés nouveaux, à l'exception des composés pour lesquels B est CO quand R est H ou 4-Cl et B est $CH_2$ quand R est H ou 4-Cl.

Pour accéder à ces thiophénols, on préconise :

(i) de condenser, en milieu basique fort, le chlorure de diméthylamino-thiocarbamoyle, de formule :

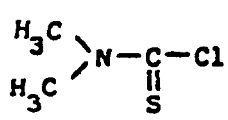

(III)

sur un phénol de formule :

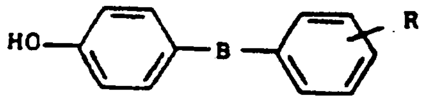

(IV)

où R et B ont les significations indiquées ci-dessus, pour obtenir un composé de formule :

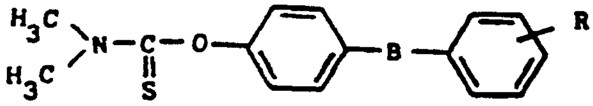

(V)

où R et B ont les significations indiquées ci-dessus,
(ii) soumettre le composé de formule V ainsi obtenu à un réarrangement de Newmann (J. Org. Chem. (1966) 31, p 3980), par chauffage, pour obtenir un composé de formule :

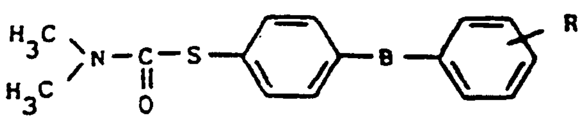

(VI)

où R et B ont les significations indiquées ci-dessus,
(iii) traiter le composé de formule VI ainsi obtenu par un alcoolate métallique, de préférence le méthanolate de sodium ou de magnésium, dans un alcool inférieur en $C_1$-$C_4$, de préférence le méthanol, pour obtenir un thiophénol de formule :

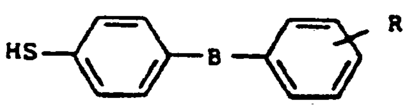

(VII)

où R et B ont les significations indiquées ci-dessus.

Selon l'invention, on propose une composition thérapeutique caractérisée en ce qu'elle renferme, en association avec un excipient physiologiquement acceptable, au moins un composé choisi parmi l'ensemble constitué par les produits de formule I et leurs épimères. Bien entendu, dans une telle composition l'ingrédient actif intervient en quantité thérapeutiquement efficace.

Les composés de formule I sont utiles en thérapeutique en tant qu'agents antithrombotiques. Ils sont notamment utiles dans la prévention et le traitement des troubles de la circulation veineuse.

Selon l'invention, on préconise l'utilisation d'une substance appartenant à l'ensemble des composés de formule I et leurs épimères pour l'obtention d'un médicament antithrombotique destiné à une utilisation thérapeutique vis-à-vis des troubles de la circulation veineuse.

D'autres caractéristiques et avantages de l'invention seront mieux compris à la lecture qui va suivre, d'exemples de préparation, nullement limitatifs, mais donnés à titre d'illustration, et, de résultats d'essais pharmacologiques. Les angles des pouvoirs rotatoires $[\alpha]_D^{20}$ sont exprimés en degrés et ont été mesurés à 20°C.

## PREPARATION I

### Obtention du diméthylthiocarbamate de O-4-(4-nitrobenzoyl)-phényle

A une suspension de 5,4 g (0,0224 mole) de (4-hydroxyphényl) (4-nitrophényl) méthanone dans 60 ml d'eau, on additionne 1,4 g (0,025 mole) de potasse en pastille. On chauffe le mélange réactionnel à 50°C pendant deux heures en agitant vigoureusement. Le mélange est ensuite ramené à 0°C et on additionne goutte à goutte une solution de 3,5 g (0.029 mole) de chlorure de diméthylthiocarbamoyle dans 15 ml de tétrahydrofuranne (T.H.F.). L'addition terminée, le mélange réactionnel est agité 15 minutes à 0°C puis une heure à 20°C. Le milieu réactionnel est ensuite hydrolysé dans 25 ml de NaOH 1N à 0°C. Le précipité obtenu est filtré et lavé à l'eau jusqu'à pH neutre. Après séchage, il est recristallisé dans un mélange chlorure de méthylène/hexane. On obtient 5,9 g (rendement : 84%) du produit attendu fondant à 168°C.

## PREPARATION II

### Obtention du diméthylthiocarbamate de S-4-(4-nitrobenzoyl)-phényle

Sous une atmosphère d'azote et sous agitation, 5 g du produit obtenu à la préparation I sont chauffés à 200-210°C pendant trois heures. La disparition du produit de départ est contrôlée par chromatographie sur couche mince en éluant avec un mélange toluène/acétate d'éthyle (4 : 9) v/v. On obtient 5 g (rendement quantitatif) du produit attendu fondant à 198-199°C.

## PREPARATION III

### Obtention du (4-mercatophényl) (4-nitrophényl) méthanone

Sous atmosphère d'azote, on dissout 9,5 g (0,030 mole) du produit obtenu à la préparation II dans 90 ml de dioxanne. On ajoute 0,039 mole de méthylate de sodium (solution à 8% dans du méthanol) et on contrôle par chromatographie sur couche mince en éluant avec un mélange hexane/acétate d'éthyle (1 : 1) v/v la disparition du produit de départ. Après une heure d'agitation à température ambiante, on acidifie le mélange réactionnel en l'hydrolysant sur une solution d'acide chlorhydrique 1N à 0°C. Le produit attendu est extrait au moyen d'acétate d'éthyle. La phase organique obtenue est lavée à l'eau jusqu'à pH neutre, séchée sur sulfate de magnésium, filtrée et le solvant est évaporé. On obtient 7,3 g (rendement : 93%) du produit attendu fondant à 116-117°C.

## PREPARATION IV

### Obtention du (4-(4-nitrobenzoyl)phényl)-2,3,4-tri-O-acétyl-1,5-dithio-β-D-xylopyranoside.

**Exemple 1a**

Un mélange de 150 ml de benzène anhydre, de 150 ml de nitrométhane et de 30 g de tamis moléculaire 0,4 nm (Commercialisé par la société dite E. MERCK) est agité à température ambiante pendant 15 minutes puis on ajoute 14,2 g (0,0553 mole) de cyanure mercurique ($Hg(CN)_2$). Après avoir agité le mélange résultant 10 minutes à température ambiante, on ajoute 19,6 g (0,0552 mole) de 2,3,4-trio-O-acétyl-1-bromo-5-thio-α-D-xylopyranoside puis 13 g (0,050 mole) de (4-mercaptophényl) (4-nitrophényl) méthanone par petites fractions. L'addition terminée, le mélange réactionnel est chauffé à 40-50°C pendant quatre heures puis filtré sur Célite® (i.e. silice diatomée pour filtration). Le résidu est lavé plusieurs fois avec de l'acétate d'éthyle. La phase organique obtenue est lavée successivement avec une solution saturée de chlorure de sodium, une solution de NaOH 1N, une solution de chlorure de sodium puis à l'eau jusqu'à pH neutre. On sèche sur sulfate de magnésium, filtre et on évapore le solvant. L'huile jaunâtre obtenue est dissoute dans 50 ml d'éther et placée 12 heures à 4°C. Le produit cristallise. Après filtration, on obtient 17,2 g du produit attendu de configuration β. Les eaux mères sont ensuite évaporées et les produits qu'elles contiennent sont séparés par "flash chromatography" en éluant avec un mélange toluène/acétate d'éthyle (8 : 1) v/v. On obtient finalement 18,6 g de l'isomère β (rendement : 70%) fondant à 166-169°C ($[\alpha]^{20}_D$ = + 92 ; C = 0,5 ($CHCl_3$)) et 3,9 g, de l'isomère α (rendement : 15%) sous forme de mousse ($[\alpha]^{20}_D$ = + 286 ; C = 0,5 ($CHCl_3$)).

**PREPARATION V**

**Obtention du (4-(4-nitrobenzoyl)phényl)-1,5-dithio-β-D-xylopyranoside.**

**Exemple 1**

Sous atmosphère d'azote, 18 g (0,0337 mole) du produit obtenu à la préparation IV (exemple 1a) sont dissous dans un mélange de 100 ml d'acétate d'éthyle et de 300 ml de méthanol, puis on ajoute 8,5 ml de méthylate de sodium en solution à 8% dans du méthanol. Après deux heures sous agitation à température ambiante, on filtre le précipité formé et on lave deux fois avec 50 ml de méthanol. Le filtrat obtenu est neutralisé au moyen de résine Amberlite® IR 120 ($H^+$) jusqu'à pH 4-5, puis après filtration, le solvant est évaporé et le résidu d'évaporation ainsi obtenu est réuni avec le précipité préalablement obtenu. On obtient 13,8 g du produit attendu (rendement quantitatif) fondant à 183°C. ($[\alpha]^{20}_D$ = + 60 ; C = 0,5 (DMSO)).

**PREPARATION VI**

**Obtention du (4-((4-nitrophényl)hydroxyméthyl)phényl)-1,5-dithio-β-D-xylopyranoside.**

**Exemple 3**

Sous atmosphère d'azote, on ajoute par petites quantités 1,2 g (0,0315 mole) de tétraborohydrure de sodium à une suspension de 11,2 g (0,0275 mole) du produit obtenu à la préparation V (exemple 1). La solution devient homogène après deux heures d'agitation à 0°C. On neutralise le milieu réactionnel au moyen de résine Amberlite® IR 120 ($H^+$) jusqu'à pH 4-5, et on évapore le solvant après filtration. Le résidu d'évaporation ainsi obtenu est purifié sur colonne de silice en éluant avec de l'acétate d'éthyle. On obtient 11,2 g (rendement quantitatif) du produit attendu fondant à 80°C. ($[\alpha]^{20}_D$ = + 8 ; C = 0,5 (méthanol)).

**PREPARATION VII**

**Obtention du (4-((4-nitrophényl)hydroxyméthyl)phény-2,3,4-tri-O-acétyl-1,5-dithio-β-D-xylopyranoside.**

**Exemple 3a**

Sous atmosphère d'azote, 7 g (0,0131 mole) de (4-(4-nitrobenzoyl)phényl)-2,3,4-tri-O-acétyl-1,5-dithio-β-D-xylopyranoside obtenu à la préparation IV (exemple 1a) sont dissous dans 70 ml de méthanol puis, à température ambiante, on additionne au mélange réactionnel 0,5 g (0,0131 mole) de tétraborohydrure de sodium. On agite pendant 30 minutes puis le milieu réactionnel est acidifié par addition de résine Amberlite® IR 120 ($H^+$) jusqu'à pH 4-5. Après filtration, on évapore le filtrat recueilli. On obtient 6,3 g du produit attendu (rendement : 90%) sous forme d'une mousse jaune. ($[\alpha]^{20}_D$ = + 29 ; C = 0,15 (méthanol)).

**PREPARATION VIII**

**Obtention du (4-(4-nitrobenzyl)phényl)-2,3,4-tri-O-acétyl-1,5-dithio-β-D-xylopyranoside.**

Sous atmosphère d'azote, 3,3 g (0,00616 mole) de (4-((4-nitrophényl) hydroxyméthyl)phényl)-2,3,4-tri-O-acétyl-1,5-dithio-β-D-xylopyranoside (exemple 3a) obtenu à la préparation VII sont mis en suspension dans 17 ml de chlorure de méthylène. Le milieu réactionnel est refroidi à 0°C puis 17 ml d'acide trifluoroacétique sont additionnés en une seule fois et 470 mg (0,0123 mole) de tétraborohydrure de sodium sont ajoutés par petites portions. L'agitation du milieu est poursuivie à 0°C pendant 1,5 h. Le milieu réactionnel est hydrolysé sur glace et extrait au chlorure de méthylène. La phase organique obtenue est lavée avec une solution saturée de bicarbonate puis à l'eau jusqu'à pH neutre. La phase organique est séchée, filtrée puis évaporée. On obtient 2,77 g (rendement : 87%) du produit attendu sous forme de mousse.

## PREPARATION IX

### Obtention du (4-(4-nitrobenzyl)phényl)-1,5-dithio-β-D-xylopyranoside

**Exemple 4**

2,79 g (0,00537 mole) de (4-(4-nitrobenzyl)phényl)-2,3,4-tri-O-acétyl-1,5-dithio-β-D-xylopyranoside obtenu à la préparation VIII sont mis en suspension dans 40 ml de méthanol puis, sous agitation à température ambiante, on ajoute 0,15 ml de méthylate de sodium en solution à 8% dans du méthanol. Après 12 heures d'agitation à température ambiante, le méthylate de sodium est neutralisé au moyen de résine Amberlite® IR 120 ($H^+$). Le milieu réactionnel est filtré, évaporé, puis le résidu d'évaporation ainsi obtenu est purifié par "flash chromatography" en éluant avec un mélange chlorure de méthylène/méthanol (95 : 5) v/v. On obtient 1,3 g du produit attendu (rendement : 60%) fondant à 163°C. ($[\alpha]^{20}_D$ = + 10 ; C = 0,5 (méthanol)).

## PREPARATION X

### Obtention du (4-(4-nitrobenzyl)phényl)-2,3,4-tri-O-acétyl-5-thio-β-D-xylopyranoside.

**Exemple 2a**

Sous atmosphère d'azote et à 3°C on mélange successivement 4,5 g (0,01965 mole) de 4-(4-nitrobenzyl)phénol, 3 ml de 2,4,6-triméthylpyridine, 70 ml de mélange toluène/nitrométhane (1 : 1) v/v et 10 g de tamis moléculaire 0,4 nm. Le milieu réactionnel est agité énergiquement pendant 20 minutes puis on introduit 5,8 g (0,0225 mole) de triflate d'argent et 8,7 g (0,0245 mole) de 1-bromo-2,3,4-tri-O-acétyl-5-thio-α-D-xylopyranoside sont ajoutés par fractions de 2,17 g toutes les 30 minutes. On agite à l'abri de la lumière et à 3°C pendant 20 heures. Le milieu réactionnel est filtré sur Célite® et on lave le précipité trois fois avec 200 ml d'acétate d'éthyle. Le filtrat obtenu est lavé avec HCl 1N puis à l'eau jusqu'à pH neutre. Après séchage sur sulfate de magnésium, filtration et évaporation, l'huile jaunâtre obtenue est purifiée par "flash chromatography" en éluant avec un mélange hexane/acétate d'éthyle. On obtient 3 g (rendement : 30%) de l'isomère β fondant à 134°C. ($[\alpha]^{20}_D$ = – 25 ; C = 0.5 ($CHCl_3$)) et 3 g de l'isomère α ($[\alpha]^{20}_D$ = + 284 ; C = 0,4 ($CHCl_3$)).

## PREPARATION XI

### Obtention du (4-(4-nitrobenzyl)phényl)-5-thio-β-D-xylopyranoside.

**Exemple 2**

Sous atmosphère d'azote et à 0°C on met 2,5 g (0,005 mole) du produit (exemple 2a) obtenu à la préparation X, en suspension dans 150 ml de méthanol puis on ajoute 0,5 ml de méthylate de sodium en solution à 8% dans du méthanol. On agite le milieu réactionnel pendant deux heures, puis on ajoute de la résine Amberlite® IR 120 (H+). Lorsque le pH neutre est atteint on évapore le méthanol sous pression réduite et on lyophilise le résidu d'évaporation ainsi obtenu. On obtient 1,9 g (rendement quantitatif) du produit attendu fondant à 166°C. ($[\alpha]^{20}_D$ = – 21 ; C = 0,5 (méthanol)).

## PREPARATION XII

### Obtention du (4-(4-nitrobenzoyl)phényl)-2,3,4-trio-O-acétyl-5-thio-β-D-xylopyranoside.

**Exemple 10a**

Sous atmosphère d'azote, on mélange successivement 1,1 g (0,0028 mole) du produit (exemple 2a) obtenu à la préparation X, 50 ml de chlorure de méthylène anhydre, 0,66 g (0,043 mole) d'oxyde de chrome ($Cr_2O_8$) et 12 ml de pyridine. Le mélange résultant est chauffé à 60°C pendant 24 heures, puis 0,66 g d'oxyde de chrome est ajouté et on poursuit le chauffage pendant 24 heures. La phase organique est séparée du résidu insoluble par décantation. Le résidu insoluble est repris par une solution de bicarbonate de sodium et d'alcool isopropylique puis extrait trois fois par du chlorure de méthylène. Les phases organiques sont réunies et lavées avec une solution de bicarbonate de sodium, à l'eau jusqu'à pH neutre, à l'acide chlorhydrique 1N, puis à l'eau jusqu'à pH neutre. On sèche sur sulfate de magnésium, filtre et évapore. Le résidu d'évaporation brut ainsi

obtenu est purifié par "flash chromatography" en éluant avec un mélange chloroforme/acétate d'éthyle (1 : 1) v/v. On obtient 0,720 g du produit de départ et 0,260 g (rendement : 24%) du produit attendu, fondant à 152°C. ($[\alpha]^{20}_D$ = – 47 ; C = 0,3 ($CHCl_3$)).

**PREPARATION XIII**

**Obtention du (4-((4-nitrophényl)hydroxyméthyl)phényl)-1,5-dithio-β-D-xylopyranoside.**

**Exemple 3**

Sous atmosphère d'azote on dissout 5,33 g (0,01 mole) du produit (exemple 1a) obtenu à la préparation IV dans 50 ml de méthanol anhydre puis 0,5 ml d'une solution de méthylate de sodium à 8% dans le méthanol sont additionnés. Le mélange est agité pendant 1 heure en contrôlant par chromatographie sur couche mince la disparition du produit de départ. Lorsque la disparition du produit de départ est totale, on additionne 0,4 mg (0,0105 mole) de tétraborohydrure de sodium ($NaBH_4$) par petites portions et on contrôle la disparition de l'intermédiaire acétylé formé précédemment. On ajoute enfin, au mélange résultant, de la résine Amberlite® IR 120 ($H^+$) pour neutraliser le milieu. Après filtration, on évapore à sec le filtrat. Le résidu d'évaporation, obtenu sous forme de mousse, est repris avec de l'eau bidistillée puis lyophilisé. On obtient 4 g du produit attendu (rendement quantitatif) fondant à 80°C. ($[\alpha]^{20}_D$ = + 8 ; C = 0,5 (méthanol)).

**PREPARATION XIV**

**Obtention du (4-((4-nitrophényl)hydroxyméthyl)phényl)-5-thio-β-D-xylopyranoside.**

**Exemple 8**

Selon le mode opératoire décrit dans la préparation XIII à partir du (4-(4-nitrobenzoyl)phényl)-2,3,4-tri-O-acétyl-5-thio-β-D-xylopyranoside obtenu à la préparation XII, on obtient avec un rendement quantitatif, le produit attendu fondant à 108-118°C. ($[\alpha]^{20}_D$ = – 7 ; C = 0,5 (méthanol)).

**PREPARATION XV**

**Obtention de la (4-mercaptophényl) (3-nitrophényl) méthanone**

Selon le mode opératoire décrit dans la préparation I, à partir de 18 g (0,07407 mole) de (4-hydroxyphényl) (3-nitrophényl) méthanone et de 12,3 g (0,0992 mole) de chlorure de diméthylthiocarbamoyle, on obtient 20,5 g (rendement : 84%) de diméthylthiocarbamate de O-4-(3-nitrobenzoyl)phényle.

Selon le mode opératoire décrit dans la préparation II, à partir de 20,5 g (0,062 mole) de diméthylthiocarbamate de O-4(3-nitrobenzoyl)phényle, on obtient 20,5 g (0,062 mole) (rendement quantitatif) de diméthylthiocarbamate de S-4-(3-nitrobenzoyl)phényle.

Selon le mode opératoire décrit dans la préparation III, à partir de 20,5 g (0,062 mole) de diméthylthiocarbamate de S-4(3-nitrobenzoyl)phényle, on obtient 15,6 g (rendement : 96%) de (4-mercaptophényl) (3-nitrophényl) méthanone fondant à 114°C.

**PREPARATION XVI**

**Obtention de la (4-cyanophényl) (4-mercaptophényl) méthanone**

Selon le mode opératoire décrit dans la préparation I, à partir de 5 g (0,0224 mole) de (4-hydroxyphényl) (3-nitrophényl) méthanone et de 3,6 g (0,0312 mole) de chlorure de diméthylthiocarbamoyle, on obtient 5,6 g (rendement : 76%) de diméthylthiocarbamate de O-4-(4-cyanobenzoyl)phényle fondant à 162°C.

Selon le mode opératoire décrit dans la préparation II, à partir de 5,2 g (0,0167 mole) de diméthylthiocarbamate de O-4(4-cyanobenzoyl)phényle, on obtient 5,2 g (rendement quantitatif) de diméthylthiocarbamate de S-4-(4-cyanobenzoyl)phényle fondant à 174°C.

Selon le mode opératoire décrit dans la préparation III, à partir de 18,6 g (0,059 mole) de diméthylthiocarbamate de S-4-(4-cyanobenzoyl)phényle, on obtient 12,5 g (rendement : 92%) de (4-mercaptophényl) (4-cyanophényl) méthanone fondant à 156°C.

**PREPARATION XVII**

**Obtention du (4-(4-cyanobenzoyl)phényl)-2,3,4-tri-O-acétyl-1,5-dithio-β-D-xylopyranoside.**

**Exemple 12a**

Selon le mode opératoire décrit dans la préparation IV, à partir de 6 g (0,0251 mole), de (4-mercaptophényl) (4-cyanophényl) méthanone obtenu à la préparation XVI, de 9,8 g (0,0276 mole) de 2,3,4-tri-O-acétyl-1-bromo-5-thio-α-D-xylopyranoside et de 7,1 g (0,0276 mole) de cyanure mercurique, on obtient 7,3 g (rendement: 52%) de l'isomère β fondant à 172°C. ($[\alpha]^{20}_D$ = + 50 ; C = 0,15 ($CHCl_3$)).

**PREPARATION XVIII**

**Obtention du (4-(4-cyanobenzoyl)phényl)-1,5-dithio-β-xylopyranoside.**

**Exemple 12**

Selon le mode opératoire décrit dans la préparation V, à partir de 2 g (0,0356 mole) du produit (exemple 12a) obtenu à la préparation XVII et de 0,75 ml de méthylate de sodium en solution à 8%, on obtient 1,38 g (rendement quantitatif) du produit attendu fondant à 164°C. ($[\alpha]^{20}_D$ = + 53 ; C = 0,197 ($CH_3OH$)).

**PREPARATION XIX**

**Obtention du (4-((4-cyanophényl)hydroxyméthyl)phényl)-1,5-dithio-β-D-xylopyranoside.**

**Exemple 13**

Selon le mode opératoire décrit dans la préparation VI, à partir de 3,7 g (0,0095 mole) du produit (exemple 12) obtenu à la préparation XVIII et de 0,370 g (0,0097 mole) de tétraborohydrure de sodium, on obtient 3 g (rendement : 81%) du produit attendu fondant à 70-85°C. ($[\alpha]^{20}_D$ = + 2,8 ; C = 0,598 ($CH_3OH$)).

**PREPARATION XX**

**Séparation des deux épimères du (4-((4-nitrophényl) hydroxyméthyl)phényl) -1,5-dithio-β-D-xylopyranoside.**

**1) Obtention du (+)-(4-((4-nitrophényl)hydroxyméthyl)phényl)-1,5-dithio-β-D-xylopyranoside.**

**Exemple 16**

11,2 g du mélange d'épimères ($[\alpha]^{20}_D$ = +8 ; C = 0,5 (méthanol)) obtenu à la préparation VI sont recristallisés dans 80 ml d'acétate d'éthyle saturé d'eau. On obtient 7,85 g de cristaux ($C_1$) ($[\alpha]^{20}_D$ = + 4 ; C = 0,4 (méthanol)) et un filtrat ($F_1$). Les cristaux ($C_1$) sont recristallisés dans 150 ml d'acétate d'éthyle contenant 1% d'eau (v/v). On obtient 3,15 g de cristaux ($C_2$) ($[\alpha]^{20}_D$ = + 17.6 ; C = 0,45 (méthanol)).

Les cristaux ($C_2$) sont recristallisés dans 40 ml d'acétate d'éthyle saturé d'eau. On obtient 1,78 g de cristaux ($C_3$) ($[\alpha]^{20}_D$ = + 23,2 ; C = 0.45 (méthanol)).

Les cristaux ($C_3$) sont à nouveau recristallisés dans 16 ml d'acétate d'éthyle saturé d'eau. On obtient 1,43 g de cristaux ($[\alpha]^{20}_D$ = + 25 ; C = 0,4 (méthanol)) de l'isomère (+) fondant à 141°C.

**2) Obtention du (–)-(4-((4-nitrophényl)hydroxyméthyl)phényl)-1,5-dithio-β-D-xylopyranoside.**

**Exemple 17**

Le filtrat (F1) est évaporé sous vide et repris à l'acétate d'éthyle contenant moins de 100 ppm d'eau. Après cristallisation, on obtient 3,9 g de cristaux ($C'_2$) ($[\alpha]^{20}_D$ = 4,6 ; C = 0,45 (méthanol)).

Les cristaux ($C'_2$) sont recristallisés dans 130 ml d'acétate d'éthyle contenant moins de 100 ppm d'eau. On obtient 1,44 g de cristaux ($C'_3$) ($[\alpha]^{20}_D$ = –10,4 ; C = 0,35 (méthanol)).

Les cristaux ($C'_3$) sont recristallisés dans 60 ml d'acétate d'éthyle contenant moins de 100 ppm d'eau. On

obtient 0,96 g de cristaux ($[\alpha]^{20}_D = -15$ ; C = 0,4 (méthanol)) de l'isomère (-) fondant de 157 à 163°C.

**PREPARATION XXI**

**Obtention de la (2-cyanophényl) (4-mercaptophényl) méthanone**

Selon le mode opératoire décrit dans la préparation I à partir de 13,3 g (0,059 mole) de (2-cyanophényl) (4-hydroxyphényl) méthanone et de 8,5 g (0,068 mole) de chlorure de diméthylthiocarbamoyle, on obtient 16,5 g (rendement : 89%) de diméthylthiocarbamate de O-4-(2-cyanobenzoyle)phényle fondant à 138°C.

Selon le mode opératoire décrit dans la préparation II, à partir de 16 g (0,052 mole) de diméthylthiocarbamate de O-4-(2-cyanobenzoyl)phényle, on obtient 10,9 g (rendement : 68%) de diméthylthiocarbamate de S-4-(2-cyanobenzoyl)phényle fondant à 112°C.

Selon le mode opératoire décrit dans la préparation II, à partir de 10,6 g (0,034 mole) de diméthylthiocarbamate de S-4-(2-cyanobenzoyl)phényle, on obtient 9 g (rendement : 80%) de (2-cyanophényl) (4-mercaptophényl) méthanone fondant à 102°C.

**PREPARATION XXII**

**Obtention de la (3-cyanophényl) (4-mercaptophényl) méthanone**

Selon le mode opératoire décrit dans la préparation I à partir de 27 g (0,121 mole) de (3-cyanophényl) (4-hydroxyphényl) méthanone et de 17,2 g (0,138 mole) de chlorure de diméthylthiocarbamoyle, on obtient 35 g (rendement : 88%) de diméthylthiocarbamate de O-4-(3-cyanobenzoyl)phényle fondant à 160°C.

Selon le mode opératoire décrit dans la préparation II à partir de 33 g (0,106 mole) de diméthylthiocarbamate de O-4-(3-cyanobenzoyl)phényle, on obtient 25 g (rendement : 79%) de diméthylthiocarbamate de S-4-(3-cyanobenzoyl)phényle fondant à 150°C.

Selon le mode opératoire décrit dans la préparation III, à partir de 22,6 g (0,073 mole) de diméthylthiocarbamate de S-4-(3-cyanobenzoyl)phényle, on obtient 16,5 g (rendement : 94,8%) de (3-cyanophényl) (4-mercaptophényl) méthanone fondant à 126°C.

De façon non limitative, on a consigné dans le tableau I ci-après un certain nombre de composés de formule I selon l'invention et dans le tableau II ci-après un certain nombre de leurs dérivés acétylés.

On a résumé dans les tableaux I et II les caractéristiques physiques des composés selon l'invention.

L'activité anti-thrombotique des produits selon l'invention a été mise en évidence selon le protocole opératoire de thrombose veineuse suivant :

on réalise une stase veineuse sous hypercoagulation selon la technique décrite par WESSLER et al. (J. Applied Physiol. 1959, p. 943-946). L'agent hypercoagulant utilisé est, comme dans la technique décrite par J. HAUPMAN et al. (Thrombosis and Haemostasis 43 (2) 1980, p. 118), une solution de facteur X activé fourni par la société dite Flow Laboratories (71 Knat pour 12,5 ml de sérum physiologique).

L'étude est réalisée sur des rats mâles Wistar, non à jeûn, de 250 à 280 g (lots de 10 animaux). Les produits à tester sont administrés per os, en suspension dans le PEG 400. Une thrombose est induite 4 heures après ce traitement et le thrombus formé est prélevé et pesé. Les résultats obtenus à la dose de 12,5 mg/kg p.o. (sauf indication contraire) ont été consignés dans le tableau III. On a également consigné dans ce tableau les résultats obtenus avec les produits connus de l'art antérieur précité.

Les produits selon l'invention présentent une activité antithrombotique veineuse 2 à 16 fois supérieure à l'activité des produits connus de l'art antérieur.

## TABLEAU I

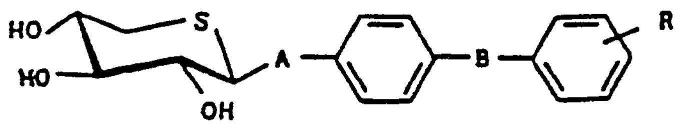

| Ex | A | B | R | F (°C) | [α]$^{20}_{D}$ (C: % p/v) |
|---|---|---|---|---|---|
| 1 | S | CO | 4-$NO_2$ | 183 | + 60 (0,5) a |
| 2 | O | $CH_2$ | 4-$NO_2$ | 166 | - 21 (0,5) b |
| 3 | S | CHOH | 4-$NO_2$ | 65 à 80 (3)(4) | + 8 (0,5) b |
| 4 | S | $CH_2$ | 4-$NO_2$ | 163 (3) | + 10 (0,5) b |
| 5 | S | CHOH | H | 160 à 190 (4) | + 11,5 (0,1) b |
| 6 | S | CHOH | 4-Cl | 169 (3)(4) | + 15,5 (0,1) b |
| 7 | S | CHOH | 3-$NO_2$ | 60 à 88 (3)(4) | + 20,3 (0,5) b |
| 8 | O | CHOH | 4-$NO_2$ | 108 à 118 (1)(3)(4) | - 7 (0,7) b |
| 9 | O | CHOH | 4-Cl | 110 à 135 (3)(4) | - 26 (0,18) b |
| 10 | O | CO | 4-$NO_2$ | 196 | - 51 (0,15) b |
| 11 | O | CO | 4-Cl | 214 | - 56 (0,15) b |
| 12 | S | CO | 4-CN | 164 | + 53 (0,197) b |
| 13 | S | CHOH | 4-CN | 70 à 85 (4) | + 2,8 (0,598) b |
| 14 | O | $CH_2$ | 4-Cl | 184 | - 45 (0,154) b |
| 15 | S | CO | 4-Cl | 160 (2) | + 50 (0,26) b |
| 16 | S | CHOH | 4-$NO_2$ | 141 | + 25 (0,4) b |
| 17 | S | CHOH | 4-$NO_2$ | 157 à 163 | - 15 (0,4) b |
| 18 | S | CO | 3-CN | 210 | + 41,2 (0,5) c |
| 19 | S | CO | 2-CN | 195 | + 59,5 (0,4) b |

**Notes :** (1) solvant résiduaire : 2,3 % $H_2O$
(2) solvant résiduaire : 2,5 % $H_2O$
(3) produit lyophilisé
(4) mélange d'épimères
(a) solvant DMSO
(b) solvant $CH_3OH$
(c) solvant THF

TABLEAU II

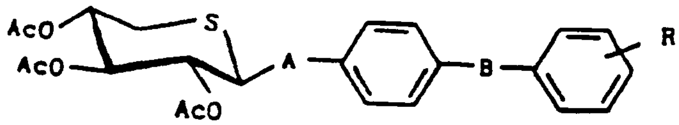

| Ex | A | B | R | F (°C) | $[\alpha]^{20}_D$ (C: % p/v) |
|---|---|---|---|---|---|
| 1a | S | CO | 4-$NO_2$ | 166 à 169 | + 92 (0,5) a |
| 2a | O | $CH_2$ | 4-$NO_2$ | 134 | - 25 (0,52) a |
| 3a | S | CHOH | 4-$NO_2$ | 74 à 98 (1) | + 29 (0,15) b |
| 5a | S | CO | H | 151 | + 93 (0,1) b |
| 7a | S | CO | 3-$NO_2$ | 142 à 144 | + 62 (0,5) a |
| 10a | O | CO | 4-$NO_2$ | 152 | - 47 (0,3) a |
| 11a | O | CO | 4-Cl | 146 | - 50 (0,17) a |
| 12a | S | CO | 4-CN | 172 | + 50 (0,15) a |
| 14a | O | $CH_2$ | 4-Cl | 112 | - 40 (0,5) a |
| 15a | S | CO | 4-Cl | 164 | +105 (0,1) b |
| 18a | S | CO | 3-CN | 144 | + 50,5 (0,54) a |
| 19a | S | CO | 2-CN | 100 à 110 | +110,4 (0,5) b |

**Notes :**

(1) mélange de diastéréoisomères
(a) solvant $CHCl_3$
(b) solvant $CH_3OH$

TABLEAU III

| PRODUIT | % INHIBITION |
|---|---|
| Ex 1 | 48 |
| Ex 2 | 76 |
| Ex 3 | 87 |
| Ex 3 a | 63 |
| Ex 4 | 72 |
| Ex 5 | 44 |
| Ex 6 | 61 |
| Ex 7 | 68 |
| Ex 8 | 80 |
| Ex 9 | 57 |
| Ex 10 | 65 |
| Ex 11 | 69 |
| Ex 12 | 69 |
| Ex 13 | 83 |
| Ex 14 | 30 |
| Ex 15 | 56 |
| Ex 16 | 72 (1) |
| Ex 17 | 66 (1) |
| Ex 18 | 31 |
| Ex 19 | 54 |
| A | 14 |
| B | 5,5 |

**Notes :**

A : produit de comparaison décrit à l'exemple 1 de EP-A-0133 103

B : produit de comparaison décrit à l'exemple 97 de EP-B-0051 023

(1) : à la dose de 7,5 mg/kg p.o.

**Revendications**

**Revendications pour les Etats Contractants suivants : AT, BE, CH, DE, FR, GB, IT, LU, NL, SE, LI**

1. Dérivé oside caractérisé en ce qu'il est choisi parmi l'ensemble comprenant
(i) les β-D-phényl-thioxylosides de formule :

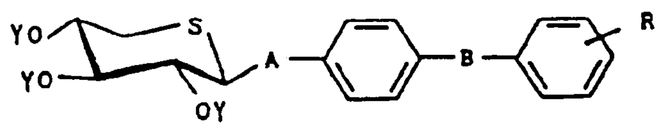

(I)

dans laquelle :
R représente un atome d'hydrogène, un atome d'halogène, un groupe nitro ou un groupe cyano,
A représente l'atome soufre ou l'atome d'oxygène,
B représente un groupe $CH_2$, CHOH ou CO,
Y représente l'atome d'hydrogène ou un groupe acyle ; et,
(ii) leurs épimères quand B est CHOH.

2. Dérivé oside selon la revendication 1, caractérisé en ce que le groupe acyle Y comporte de 2 à 5 atomes de carbone et représente notamment le groupe $CH_3CO$.

3. (4-((4-nitrophényl)hydroxyméthyl)phényl)-1,5-dithio-β-D-xylopyranoside.

4. (4-(4-cyanobenzoyl)phényl)-1,5-dithio-β-D-xylopyranoside.

5. (4-((4-cyanophényl)hydroxyméthyl)phényl)-1,5-dithio-β-D-xylopyranoside.

6. Composition thérapeutique caractérisée en ce qu'elle renferme, en association avec un excipient physiologiquement acceptable, au moins un dérivé oside choisi parmi l'ensemble comprenant les β-D-phényl-thioxylosides de formule I et leurs épimères selon la revendication 1.

7. Utilisation d'une substance appartenant à l'ensemble des β-D-phényl-thioxylosides de formule I et de leurs épimères selon la revendication 1, pour l'obtention d'un médicament antithrombotique destiné à une utilisation thérapeutique vis-à-vis des troubles de la circulation veineuse.

8. Procédé de préparation d'un β-D-phényl-thioxyloside de formule I selon la revendication 1 caractérisé en ce que :
(i) on fait réagir un composé de formule :

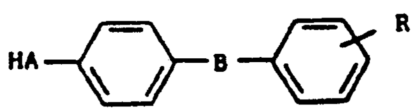

(II)

où A, B et R sont définis comme ci-dessus,
avec un dérivé du thioxylose choisi parmi l'ensemble comprenant les haloacylthioxylosides et acylthioxylosides de formules :

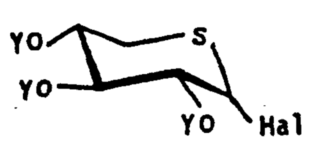

(VIIIa)

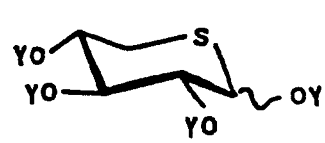

(VIIIb)

dans lesquelles Hal représente un atome d'halogène tel que Cl ou Br (l'atome de brome étant ici l'atome d'halogène préféré) et Y représente un groupe acyle, notamment un groupe acyle aliphatique, comprenant

un nombre total d'atomes de carbone de 2 à 5 et de préférence le groupe acétyle, dans un solvant inerte à raison de 1 mole de II pour environ 1,1 à 1,2 moles de dérivé du thioxylose, en présence d'un accepteur d'acide ou d'un acide de Lewis et,

(ii) si nécessaire, on procède à une réaction de désacylation à une température comprise entre la température ambiante (15-25°C) et la température de reflux du milieu réactionnel, dans un alcool inférieur en $C_1$-$C_4$ (de préférence le méthanol) en présence d'un alcoolate métallique (de préférence le méthylate de magnésium ou le méthylate de sodium).

9. Procédé selon la revendication 8, caractérisé en outre par le fait que le composé de formule II où A est S, intervenant au stade (i) est préparé selon les étapes suivantes :

a) condensation, en milieu basique fort du chlorure de diméthylaminothiocarbamoyle de formule :

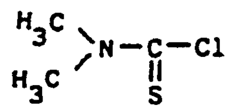

(III)

avec un phénol de formule :

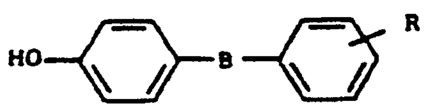

(IV)

où R et B ont les significations indiquées ci-dessus, pour obtenir un composé de formule :

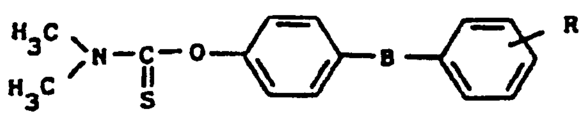

(V)

où R et B sont définis comme ci-dessus,

b) réarrangement du composé de formule V ainsi obtenu par chauffage, pour obtenir un composé de formule :

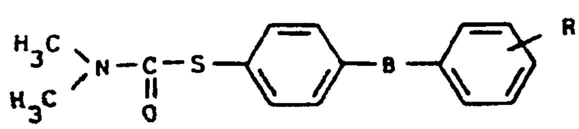

(VI )

où R et B sont définis comme indiqués indiqués ci-dessus et,

c) traitement du composé de formule VI ainsi obtenu par un alcoolate métallique, de préférence le méthanolate de sodium ou de magnésium, dans un alcool inférieur en $C_1$-$C_4$, de préférence le méthanol, pour obtenir un thiophénol de formule :

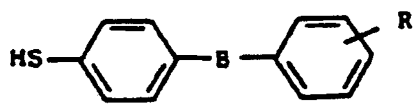

(VII )

où R et B sont définis comme indiqué ci-dessus.

10. Produit intermédiaire nouveau intervenant dans la synthèse des β-D-phényl-thioxylosides de formule I selon la revendication 1, caractérisé en ce qu'il est choisi parmi les thiophénols de formule :

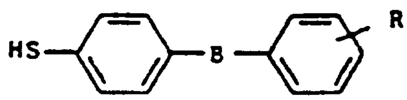

dans laquelle B et R sont définis comme indiqués ci-dessus avec la condition supplémentaire que R soit différent de H et 4-Cl quand B est CO ou $CH_2$.

**Revendications pour les Etats contractants suivants : ES, GR**

1. Procédé de préparation d'un composé β-D-phényl-thioxyloside de formule

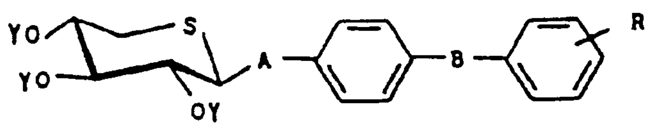

(I)

dans laquelle
R représente un atome d'hydrogène, un atome d'halogène, un groupe nitro ou un groupe cyano,
A représente l'atome soufre ou l'atome d'oxygène,
B représente un groupe $CH_2$, CHOH ou CO,
Y représente l'atome d'hydrogène ou un groupe acyle ; et des épimères correspondants quand B est CHOH, ledit procédé étant caractérisé en ce que :
(i) on fait réagir un composé de formule :

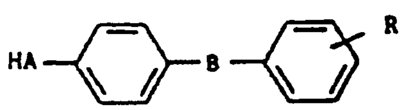

(II)

où A, B et R sont définis comme ci-dessus,
avec un dérivé du thioxylose choisi parmi l'ensemble comprenant les haloacylthioxylosides et acylthioxylosides de formules :

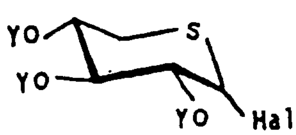

(VIIIa)

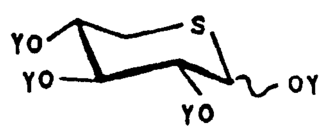

(VIIIb)

dans lesquelles Hal représente un atome d'halogène tel que Cl ou Br (l'atome de brome étant ici l'atome d'halogène préféré) et Y représente un groupe acyle, notamment un groupe acyle aliphatique, comprenant un nombre total d'atomes de carbone de 2 à 5 et de préférence le groupe acétyle, dans un solvant inerte à raison de 1 mole de II pour environ 1,1 à 1,2 moles de dérivé du thioxylose, en présence d'un accepteur d'acide ou d'un acide de Lewis et,
(ii) si nécessaire, on procède à une réaction de désacylation à une température comprise entre la température ambiante (15-25°C) et la température de reflux du milieu réactionnel, dans un alcool inférieur en $C_1$-$C_4$ (de préférence le methanol) en présence d'un alcoolate métallique (de préférence le méthylate de magnésium ou le méthylate de sodium).

2. Procédé selon la revendication 1, caractérisé en outre par le fait que le composé de formule II où A est S, intervenant au stade (i) est préparé selon les étapes suivantes :
a) condensation, en milieu basique fort du chlorure de diméthylaminothiocarbamoyle de formule :

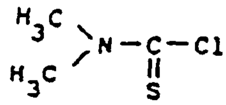

(III)

avec un phénol de formule

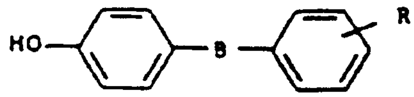

(IV)

où R et B ont les significations indiquées ci -dessus, pour obtenir un composé de formule :

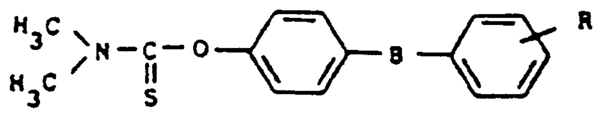

(V)

où R et B sont définis comme ci-dessus,
b) réarrangement du composé de formule V ainsi obtenu par chauffage, pour obtenir un composé de formule :

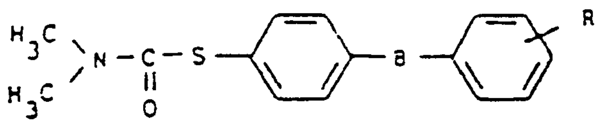

(VI)

où R et B sont définis comme indiqués ci-dessus et,
c) traitement du composé de formule VI ainsi obtenu par un alcoolate métallique, de préférence le méthanolate de sodium ou de magnésium, dans un alcool inférieur en $C_1$-$C_4$, de préférence le méthanol, pour obtenir un thiophénol de formule :

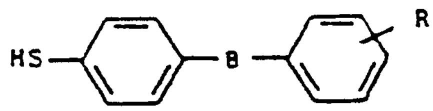

(VII)

où R et B sont définis comme indiqué ci-dessus.

## Claims

### Claims for the following Contracting States : AT, BE, CH, DE, FR, GB, IT, LI, LU, NL, SE

1. An oside derivative which is selected from the group consisting of :
(i) the β-D-phenylthioxylosides of the formula :

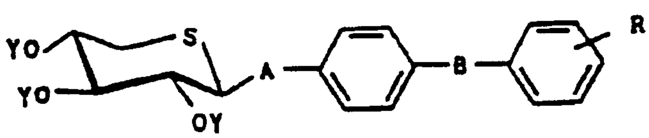

(I)

in which :
R represents a hydrogen atom, a halogen atom, a nitro group or a cyano group,
A represents the sulfur atom or the oxygen atom,

B represents a $CH_2$, CHOH or CO group and
Y represents the hydrogen atom or an acyl group ; and
(ii) epimers thereof when B is CHOH.

2. An oside derivative according to claim 1, wherein the acyl group Y contains from 2 to 5 carbon atoms and represents the group $CH_3CO$ in particular.

3. (4-((4-Nitrophenyl)hydroxymethyl)phenyl)-1,5-dithio-β-D-xylopyranoside.

4. (4-(4-Cyanobenzoyl)phenyl)-1,5-dithio-β-D-xylopyranoside.

5. (4-((4-Cyanophenyl)hydroxymethyl)phenyl)-1,5-dithio-β-D-xylopyranoside.

6. A therapeutic composition which contains, in association with a physiologically acceptable excipient, at least one oside derivative selected from the group consisting of the β-D-phenylthioxylosides of the formula I and epimers thereof according to claim 1.

7. Use of a substance belonging to the group of the β-D-phenylthioxylosides of the formula I and epimers thereof according to claim 1 for the preparation of an antithrombotic drug to be used in therapy for the treatment of disorders of the venous circulation.

8. A method for the preparation of a β-D-phenylthioxyloside of the formula I according to claim 1, wherein:
(i) a compound of the formula :

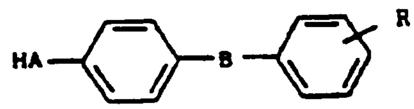

(II)

in which A, B and R are defined as above,
is reacted with a thioxylose derivative selected from the group consisting of the halogenoacylthioxylosides and acylthioxylosides of the formulae :

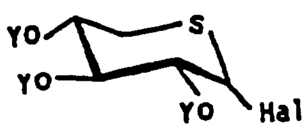

(VIIIa)

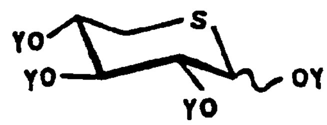

(VIIIb)

in which Hal represents a halogen atom, such as C1 or Br (the bromine atom being the preferred halogen atom here), and Y represents an acyl group, especially an aliphatic acyl group containing a total of 2 to 5 carbon atoms and preferably the acetyl group, in an inert solvent, at a rate of 1 mol of II to about 1.1 to 1.2 mol of thioxylose derivative, in the presence of an acid acceptor or a Lewis acid, and
(ii) if necessary, a deacylation reaction is carried out at a temperature between room temperature (15-25°C) and the reflux temperature of the reaction medium, in a $C_1$-$C_4$ lower alcohol (preferably methanol), in the presence of a metal alcoholate (preferably magnesium methylate or sodium methylate).

9. The method according to claim 8, wherein the compound of the formula II in which A is S, which is used in stage (i), is prepared according to the following steps :
a) condensation, in a strong basic medium, of dimethylaminothiocarbamoyl chloride of the formula :

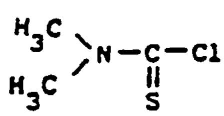

(III)

with a phenol of the formula :

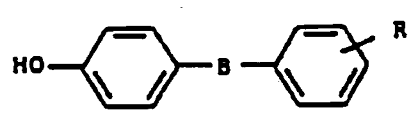

(IV)

in which R and B have the meanings indicated above, to give a compound of the formula :

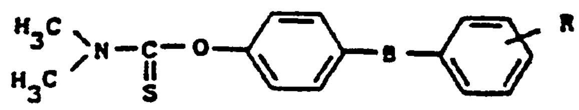

(V)

in which R and B are defined as above,

b) rearrangement of the resulting compound of the formula V, by heating, to give a compound of the formula:

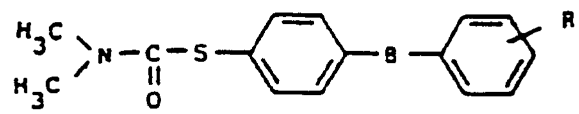

(VI)

in which R and B are defined as indicated above, and

c) treatment of the resulting compound of the formula VI with a metal alcoholate, preferably sodium or magnesium methanolate, in a $C_1$-$C_4$ lower alcohol, preferably methanol, to give a thiophenol of the formula :

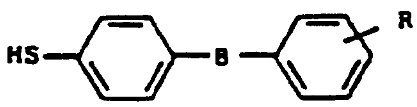

(VII)

in which R and B are defined as indicated above.

10. A novel intermediate used in the synthesis of the β-D-phenylthioxylosides of the formula I according to claim 1, which is selected from the thiophenols of the formula :

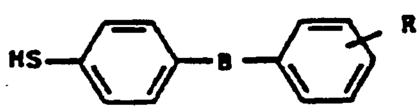

in which B and R are defined as indicated above, with the additional proviso that R is different from H and 4-C1 when B is CO or $CH_2$.

**Claims fo the following Contracting States : ES, GR**

1. A method for the preparation of a β-D-phenylthioxyloside compound of the formula :

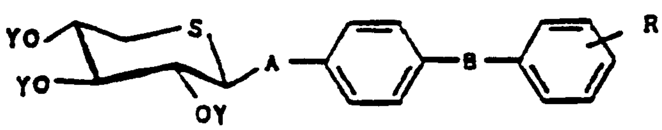

(I)

in which

R represents a hydrogen atom, a halogen atom, a nitro group or a cyano group,

A represents the sulfur atom or the oxygen atom,

B represents a $CH_2$, CHOH or CO group and

Y represents the hydrogen atom or an acyl group ; and epimers thereof when B is CHOH, wherein

(i) a compound of the formula

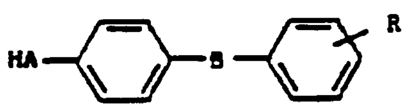

(II)

in which A, B and R are defined as above,
is reacted with a thioxylose derivative selected from the group consisting of the halogenoacylthioxylosides and acylthioxylosides of the formulae :

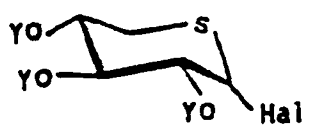

(VIIIa)

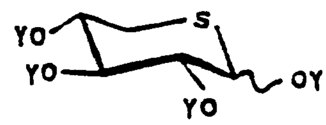

(VIIIb)

in which Hal represents a halogen atom, such as C1 or Br (the bromine atom being the preferred halogen atom here), and Y represents an acyl group, especially an aliphatic acyl group containing a total of 2 to 5 carbon atoms and preferably the acetyl group, in an inert solvent, at a rate of 1 mol of II to about 1.1. to 1.2. mol of thioxylose derivative, in the presence of an acid acceptor or a Lewis acid, and
(ii) if necessary, a desacylation reaction is carried out at a temperature between room temperature (15-25°C) and the reflux temperature of the reaction medium, in a $C_1$-$C_4$ lower alcohol (preferably methanol), in the presence of a metal alcoholate (preferably magnesium methylate or sodium methylated).

2. The method according to claim 1, wherein the compound of the formula II in which A is S, which is used in stage (i), is prepared according to the following steps :

a) condensation, in a strong basic medium, of dimethylaminothiocarbamoyl chloride of the formula :

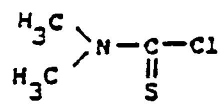

(III)

with a phenol of the formula :

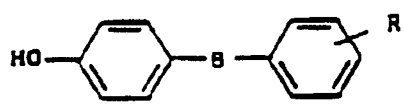

(IV)

in which R and B have the meanings indicated above, to give a compound of the formula :

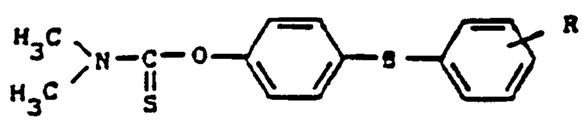

(V)

in which R and B are defined as above,

b) rearrangement of the resulting compound of the formula V, by heating, to give a compound of the formula:

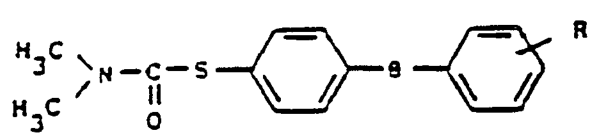

(VI)

in which R and B are defined as indicated above, and

c) treatment of the resulting compound of the formula VI with a metal alcoholate, preferably sodium or magnesium methanolate, in a $C_1$-$C_4$ lower alcohol, preferably methanol, to give a thiophenol of the formula :

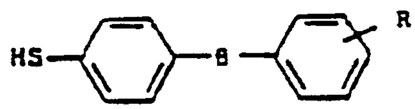

(VII)

in which R and B are defined as indicated above.

## Patentansprüche

### Patentansprüche für folgende Vertragsstaaten : AT, BE, CH, DE, FR, GB, IT, LU, NL, SE, LI

1. Osid-Derivat, dadurch gekennzeichnet, daß es aus der Gruppe ausgewählt wird, die umfaßt
(i) die β-D-Phenyl-thioxyloside der Formel

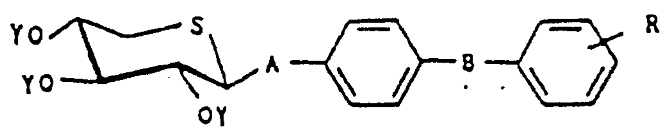

(I)

in der
R ein Wasserstoffatom, ein Halogenatom, eine Nitrogruppe oder eine Cyanogruppe darstellt,
A ein Schwefelatom oder ein Sauerstoffatom bedeutet,
B eine Gruppe $CH_2$, CHOH oder CO ist, und
Y ein Wasserstoffatom oder eine Acylgruppe darstellt ; und
(ii) ihre Epimeren, wenn B CHOH ist.

2. Derivat nach Anspruch 1, dadurch gekennzeichnet, daß die Acylgruppe Y 2-5 Kohlenstoffatome umfaßt und insbesondere die Gruppe $CH_3CO$ darstellt.

3. (4-((4-Nitrophenyl)hydroxymethyl)phenyl)-1,5-dithio-β-D-xylopyranosid.

4. (4-((4-Cyanophenyl)phenyl)-1,5-dithio-β-D-xylopyranosid.

5. (4-((4-Cyanophenyl)hydroxymethyl)phenyl)-1,5-dithio-β-D-xylopyranosid.

6. Therapeutische Zusammensetzung, dadurch gekennzeichnet, daß sie in Verbindung mit einem physiologisch akzeptablen Bindemittel mindestens ein Osid-Derivat ausgewählt aus der Einheit enthält, die die β-D-Phenyl-thioxyloside der Formel I und ihre Epimeren nach Anspruch 1 enthält.

7. Verwendung einer Substanz aus der Gruppe der β-D-Phenyl-thioxyloside der Formel I und ihren Epimeren gemäß Anspruch 1 zur Herstellung eines antithrombotischen Medikamentes, das für die therapeutische Anwendung gegenüber venösen Kreislaufstörungen vorgesehen ist.

8. Verfahren zur Herstellung eines β-D-Phenyl-thioxylosides der Formel I nach Anspruch 1, dadurch gekennzeichnet, daß man
(i) eine Verbindung der Formel

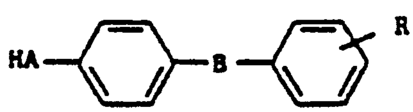

(II)

worin A, B und R wie oben definiert sind, mit einem Thioxylose-Derivat, ausgewählt aus der die Haloacylthiocyloside und die Acylthiocyloside der Formeln

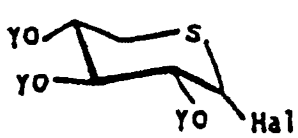

(VIIIa)

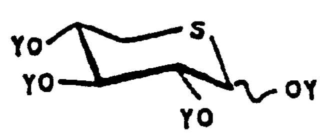

(VIIIb)

worin Hal ein Halogenatom wie C1 oder Br (das Bromatom ist hier das bevorzugte Halogenatom) und Y eine Acylgruppe, insbesondere eine aliphatische Acylgruppe mit einer Gesamtanzahl von 2 bis 5 Kohlenstoffatomen, und bevorzugt die Acetylgruppe darstellen, umfassenden Gruppe zur Reaktion bringt, in einem inerten Lösungsmittel, im Verhältnis von 1 Mol der Verbindung II zu etwa 1,1 bis 1,2 Mol des Thioxylose-Derivates und in Anwesenheit eines Säureakzeptors oder einer Lewis-Säure, und

(ii) wenn erforderlich, eine Deacylierungs-Reaktion bei einer Temperatur zwischen der Umgebungstemperatur (15-20°C) und der Rückflußtemperatur der Reaktionsmischung in einem niederen Alkohol mit 1 bis 4 Kohlenstoffatomen (vorzugsweise Methanol) in Anwesenheit eines Metall-Alkoholats (vorzugsweise Magnesiummethylat oder Natriummethylat) durchführt.

9. Verfahren nach Anspruch 8, dadurch gekennzeichnet, daß die Verbindung der Formel II, worin A Schwefel ist und die in Stufe (i) eingreift, gemäß den folgenden Stufen hergestellt wird :

a) Kendensation von Dimethylaminothiocarbamoylchlorid der Formel

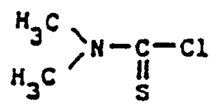

(III)

im stark basischen Medium mit einem Phenol der Formel

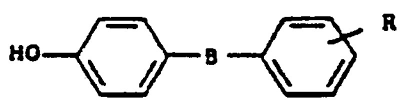

(IV)

worin R und B die oben angegebenen Bedeutungen besitzen, um eine Verbindung der Formel

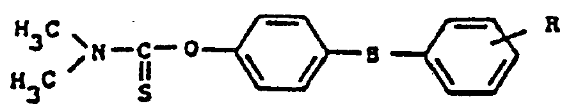

(V)

zu erhalten, in der R und B wie oben angegeben definiert sind,

b) Umlagerung der auf diese Weise erhaltenen Verbindung der Formel V durch Erhitzung, um eine Verbindung der Formel

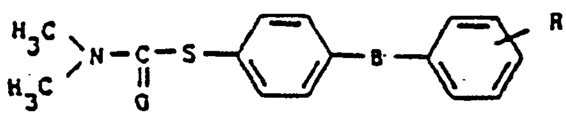

(VI )

zu erhalten, in der R und B wie oben angegeben definiert sind,

c) Behandlung der auf diese Weise erhaltenen Verbindung der Formel VI mit einem Metall-Alkoholat, vorzugsweise Natrium- oder Magnesium-Methanolat, in einem niederen Alkohol mit 1 bis 4 Kohlenstoffatomen, vorzugsweise Methanol, um ein Thiophenol der Formel

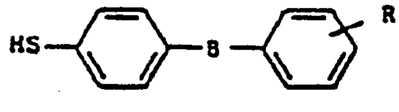

(VII )

zu erhalten, worin R und B wie oben angegeben definiert sind.

10. Neues Zwischenprodukt, das in die Synthese der β-D-Phenyl-thioxyloside der Formel I nach Anspruch 1 eingreift, dadurch gekennzeichnet, daß es ausgewählt ist aus den Thiophenolen der Formel

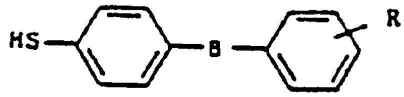

worin B und R wie oben angegeben definiert sind, unter der zusätzlichen Bedingung, daß R anders als H und 4-C1 ist, wenn B entweder CO oder $CH_2$ ist.

**Patentansprüche für folgende Vertragsstaaten : ES, GR**

1. Verfahren zur Herstellung neuer β-D-Phenyl-thio-xyloside-Verbindung der Formel

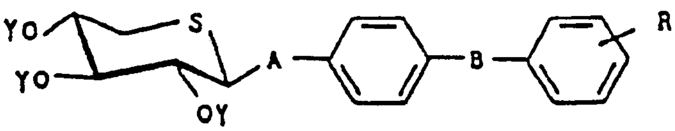

(I)

in der

R ein Wasserstoffatom, ein Halogenatom, eine Nitrogruppe oder eine Cyanogruppe darstellt,
A ein Schwefelatom oder ein Sauerstoffatom bedeutet,
B eine Gruppe $CH_2$, CHOH oder CO ist,
Y ein Wasserstoffatom oder eine Acylgruppe darstellt ; und ihre Epimeren, wenn B CHOH ist, dadurch gekennzeichnet, daß man

(i) eine Verbindung der Formel

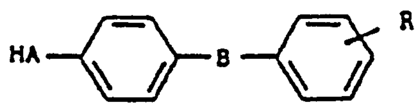

(II)

worin A, B und R wie oben definiert sind, mit einem Thioxylose-Derivat, ausgewählt aus der die Haloacylthiocyloside und die Acylthiocyloside der Formeln

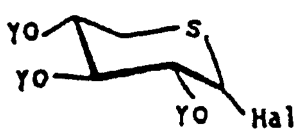

(VIIIa)

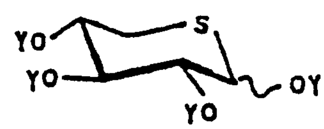

(VIIIb)

worin Hal ein Halogenatom wie C1 oder Br (das Bromatom ist hier das bevorzugte Halogenatom) und Y eine Acylgruppe, insbesondere eine aliphatische Acylgruppe mit einer Gesamtanzahl von 2 bis 5 Kohlenstoffatomen, und bevorzugt die Acetylgruppe darstellen, umfassenden Gruppe zur Reaktion bringt, in einem inerten Lösungsmittel, im Verhältnis von 1 Mol der Verbindung II zu etwa 1,1 bis 1,2 Mol des Thioxylose-Derivates und in Anwesenheit eines Säureakzeptors oder einer Lewis-Säure, und

(ii) wenn erforderlich, eine Deacylierungs-Reaktion bei einer Temperatur zwischen der Umgebungstemperatur (15-20°C) und der Rückflußtemperatur der Reaktionsmischung in einem niederen Alkohol mit 1 bis 4 Kohlenstoffatomen (vorzugsweise Methanol) in Anwesenheit eines Metall-Alkoholats (vorzugsweise Magnesiummethylat oder Natriummethylat) durchführt.

2. Verfahren nach Anspruch 1, dadurch gekennzeichnet, daß die Verbindung der Formel II, worin A Schwefel ist und die in Stufe (i) eingreift, gemäß den folgenden Stufen hergestellt wird :

a) Kondensation von Dimethylaminothiocarbamoylchlorid der Formel

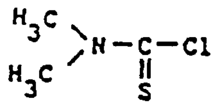

(III)

im stark basischen Medium mit einem Phenol der Formel

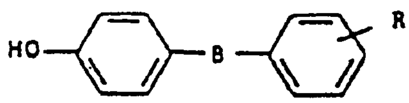

(IV)

worin R und B die oben angegebenen Bedeutungen besitzen, um eine Verbindung der Formel

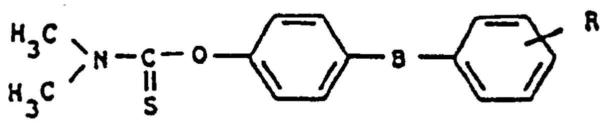

(V)

zu erhalten, in der R und B wie oben angegeben definiert sind,

b) Umlagerung der auf diese Weise erhaltenen Verbindung der Formel V durch Erhitzung, um eine Verbindung der Formel

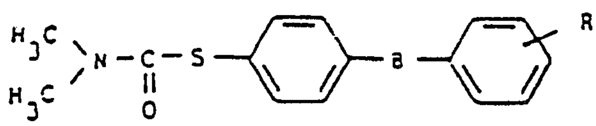

(VI)

zu erhalten, in der R und B wie oben angegeben definiert sind,

c) Behandlung der auf diese Weise erhaltenen Verbindung der Formel VI mit einem Metall-Alkoholat, vorzugsweise Natrium- oder Magnesium-Methanolat, in einem niederen Alkohol mit 1 bis 4 Kohlenstoffatomen, vorzugsweise Methanol, um ein Thiophenol der Formel

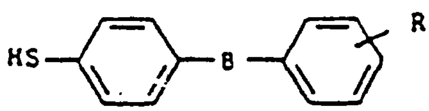

(VII)

zu erhalten, worin R und B wie oben angegeben definiert sind.